# EUROPEAN PATENT SPECIFICATION

(11) **EP 2 379 143 B1**
(45) Date of publication and mention of the grant of the patent: **20.08.2014**
(21) Application number: 09801779.1
(22) Date of filing: 21.11.2009
(51) Int. Cl.: A61M 15/00, A61M 16/06, A61M 16/20, F16K 15/16

(54) **VALVED HOLDING CHAMBER AND MASK THEREFOR**
HALTEKAMMER MIT VENTIL UND MASKE DAFÜR
CHAMBRE DE RETENUE À SOUPAPE ET MASQUE POUR CELLE-CI

(30) Priority: 18.12.2008 US 138541 P
(43) Date of publication of application: 26.10.2011
(73) Proprietor: Koninklijke Philips N.V., 5656 AE Eindhoven (NL)
(72) Inventor: VON HOLLEN, Dirk, Briarcliff Manor New York 10510-8001 (US); DENYER, Jonathan, Stanley, Harold, Briarcliff Manor New York 10510-8001 (US); LIEBERMAN, Eric, Briarcliff Manor New York 10510-8001 (US)
(74) Representative: van Velzen, Maaike Mathilde
(86) International application number: PCT/IB2009/055257
(87) International publication number: WO 2010/070496

(56) References cited:
- EP-A2- 1 854 493
- WO-A2-02/092146
- US-A- 5 036 840
- US-A- 5 297 543
- US-A- 5 385 140
- US-A- 6 039 042
- US-A1- 2002 195 108
- US-A1- 2004 216 735
- US-A1- 2007 283 962

## Description

The present invention pertains to respiratory drug delivery systems, and, in particular, to various embodiments of an improved valved holding chamber and a mask for use with a respiratory drug delivery apparatus such as, without limitation, a valved holding chamber.

It is well known to deliver a medication to a patient's respiratory system to treat a medical condition using a respiratory drug delivery apparatus. For example, a patient suffering from an acute asthmatic attack may use a respiratory drug delivery apparatus to deliver a bronchodilator, such as albuterol (salbutamol), in the form of a fine mist to the patient's respiratory system.

A conventional a respiratory drug delivery apparatus often consists of a metered dose inhaler ("MDI") and a spacer or valved holding chamber. The MDI, also known simply as an "inhaler", includes a canister or nebulizer that contains the medication under pressure and a canister holder, which is typically "L" shaped. Although it is common for a patient to use the canister holder as a mouthpiece for receiving the aerosolized medication into their airway directly from the aerosol dispensing leg of the canister holder, this configuration may not optimize the mixing of the medication with the air because the aerosolized medication is injected directly into the airway. Without adequate mixing of the drug with the air, the medication may not be inhaled into the patient's lungs where it is effective, but instead may form as droplets that are deposited in the patient's mouth and swallowed without the desired medicinal effect.

To enhance mixing of the medication with air, it is known to provide a spacer, also commonly referred to as a valved holding chamber, which attaches to the aerosol dispending end (the outlet end) of the canister holder. The spacer, which is typically a small hollow cylinder with a one-way valve at the downstream end, receives the aerosol from the canister and allows it to form into a fine mist for inhalation into the airway of the patient. Optionally, a mask may be provided at the end of the spacer opposite the MDI so that the patient can breath through his or her mouth to receive the medication. Examples of conventional spacers and associated components are shown in U.S. Pat. Nos. 4,470,412; 4,809,692; and 4,832,015 all to Nowacki et al.; U.S. Pat. No. 5,012,803 to Foley et al.; U.S. Pat. No. 5,042,467 to Foley; U.S. Pat. No. 5,385,140 to Smith, U.S. Pat. No. 5,848,599 to Foley et al., and U.S. Pat. No. 6,557,549 to Schmidt et al.

While the spacers described in these patents improve mixing of the medication with air, still further improvements in a respiratory drug delivery apparatus design are desirable, particularly those that help to enhance treatment by encouraging proper use thereof, such as proper use of an MDI and spacer by the patient.

The invention is defined by independent claim 1. In one embodiment, the invention provides a valved holding chamber that includes a main chamber housing and a mouthpiece assembly coupled to a first end of the main chamber housing. The mouthpiece assembly includes a dual flap exhalation valve element providing a one-way flow of gas from within the mouthpiece assembly to ambient atmosphere. The exhalation valve element comprises a first flap having a first gas flow resistance and a second flap having a second gas flow resistance. The first resistance is less that the second resistance such that when a first exhalation gas flow having a gas flow rate that is less than or equal to a threshold gas flow rate is present within the mouthpiece assembly, the first flap exhibits a first degree of movement as a result of said first exhalation gas flow which is greater than a second degree of movement exhibited by the second flap as a result of the first exhalation gas flow. Preferably, the first and second gas flow resistances are such that when a second exhalation gas flow having a gas flow rate that is greater than the threshold gas flow rate is present within the mouthpiece assembly, the first flap exhibits a third degree of movement as a result of the second exhalation gas flow and the second flap exhibits a fourth degree of movement as a result of the second exhalation gas flow, the third degree of movement and the fourth degree of movement being substantially the same. In one particular embodiment, the second gas flow resistance of the second flap is such that the second degree of movement is no movement.

The cross-sectional area of the first flap of the exhalation valve element may be greater than the cross sectional area of the second flap of the exhalation valve element. Also, the thickness of the first flap of the exhalation valve element may be less than the thickness of the second flap of the exhalation valve element.

The mouthpiece assembly may include a first exhalation port and a second exhalation port, with the first exhalation port being larger than the second exhalation port. In this embodiment, the first flap is positioned to cover the first exhalation port and the second flap is positioned to cover the second exhalation port.

The exhalation valve element may comprise a single piece, wherein the first flap is connected to the second flap. Furthermore, at least a portion of the first flap and at least a portion of the second flap may positioned directly adjacent to one another separated by an elongated slit. Alternatively, one of the first flap and the second flap may surround the other of the first flap and the second flap on three sides thereof.

In still another particular embodiment, the main chamber housing is generally cylindrically shaped, and the mouthpiece assembly includes a main body portion and a mouthpiece portion. The main body portion has a plurality of legs which support the valved holding chamber in a first orientation and which prevent the valved holding chamber from freely rolling. In addition, the main body portion may include a tether holding peg positioned between a first one of the legs and a second one of the legs, and the tether holding peg may be recessed with respect to the bottom of legs.

In another example, the specification describes a valved holding chamber that includes a main chamber housing and an MDI adapter coupled to an end of the main chamber housing, wherein the MDI adapter has a rigid outer portion for coupling the MDI adapter to the end of the main chamber housing and a flexible inner portion structured to receive and hold an outlet of an MDI. The rigid outer portion may include a fluid flow actuated noisemaker, such as a whistle or a noisemaker including a sound reed. Preferably, the rigid outer portion includes at least one support member, such as an arcuate engagement surface, structured to directly or indirectly engage at least a portion of the outer periphery of the outlet of the MDI. The at least one support member may comprise a first support member and a second support member positioned opposite the first support member.

According to yet another embodiment, the invention provides an assembly comprising a mask for a respiratory drug delivery apparatus and a valved holding chamber. A main body of the mask comprises a dual flap exhalation valve element substantially as described above. In still another example, a valved holding chamber is described that includes a main chamber housing and an MDI adapter coupled to an end of the main chamber housing. At least a portion of the MDI adapter comprises a flexible portion having an inner surface defining an opening for receiving an outlet of an MDI. The inner surface has a plurality of ribs extending therefrom, wherein the ribs are each structured to engage a respective portion of the outlet of the MDI in order to reduce the friction forces that are applied to the MDI.

In still another example, a valved holding chamber is described that includes a main chamber housing and an MDI adapter coupled to an end of the main chamber housing. At least a portion of the MDI adapter comprises a flexible portion having an opening for receiving an outlet of an MDI. The flexible portion further includes an automatic protective closure mechanism coupled to the opening. The automatic protective closure mechanism has an open condition and a closed condition, wherein the automatic protective closure mechanism is structured to move from the closed condition to the open condition in response to a force being applied to the automatic protective closure mechanism (e.g.,. by the MDI being inserted therein). In one particular example, the automatic protective closure mechanism is a duckbill type valve. In another particular example, the automatic protective closure mechanism is a plurality of self closing flaps.

Therefore, it should now be apparent that the invention substantially achieves all the above aspects and advantages. Additional aspects and advantages of the invention will be set forth in the description that follows, and in part will be obvious from the description, or may be learned by practice of the invention. Moreover, the aspects and advantages of the invention may be realized and obtained by means of the instrumentalities and combinations particularly pointed out in the appended claims.

The accompanying drawings illustrate presently preferred embodiments of the invention and examples, and together with the general description given above and the detailed description given below, serve to explain the principles of the invention. As shown throughout the drawings, like reference numerals designate like or corresponding parts.
Figures 1, 2 and 3 are isometric views of a valved holding chamber according to one embodiment of the present invention;
Figures 4 and 5 are isometric views of the main chamber housing forming a part of the valved holding chamber shown in Figures 1-3;
Figures 6 and 7 are side elevational and front elevational views, respectively, of the main chamber housing forming a part of the valved holding chamber shown in Figures 1-3;
Figure 8 is a top plan view of the valve disk forming a part of the valved holding chamber shown in Figures 1-3;
Figure 9 is a front elevational view of the main chamber housing having the valve disk attached thereto;
Figure 10 is a front elevational view of the mouthpiece assembly forming a part of the valved holding chamber shown in Figures 1-3;
Figures 11, 12 and 13 are isometric, front elevational and rear elevational views, respectively, of the main body portion of the mouthpiece assembly shown in Figure 10;
Figure 14 is a top plan view of the dual flap exhalation valve forming a part of the valved holding chamber shown in Figures 1-3;
Figure 15 is a front elevational view of the valved holding chamber shown in Figures 1-3;
Figures 16 and 17 are side elevational views of the valved holding chamber shown in Figures 1-3 holding different types of MDIs;
Figures 18 and 19 are front and rear elevational views, respectively, of the two-part MDI adapter forming a part of the valved holding chamber shown in Figures 1-3;
Figures 20, 21, 22 and 23 are front and rear elevational and isometric views, respectively, of the end cap of the two-part MDI adapter shown in Figures 18 and 19;
Figures 24, 25, 26 and 27 are front and rear elevational and isometric views, respectively, of the inner portion of the two-part MDI adapter shown in Figures 18 and 19;
Figure 28 is a rear elevational view of the valved holding chamber shown in Figures 1-3;
Figures 29 and 30 are front elevational and isometric views, respectively, of the MDI adapter holding an MDI having an oblong outlet;
Figures 31 and 32 are front elevational and isometric views, respectively, of the MDI adapter holding an MDI having an round outlet;
Figures 33-39 are isometric views of alternative mouthpiece assemblies having alternative exhalation valve elements that may be used in a valved holding chamber;
Figures 40 and 41 are isometric views of a mask that may be used in connection with a valved holding chamber according to an arrangement of the invention;
Figure 42 is a front isometric view and Figure 43 is a rear isometric view of a two-part MDI adapter according to an example;
Figures 44 and 45 are front and rear isometric views, respectively, and Figure 46 is a front elevational view of the flexible inner portion of the example shown in Figures 42 and 43;
Figure 47 is a rear isometric view of the two-part MDI adapter of Figures 42 and 43 showing the duckbill type valve in its open condition (without an MDI), and Figure 48 is a rear isometric view of the two-part MDI adapter of Figures 42 and 43 showing the duckbill type valve in its open condition with an MDI inserted therein; and
Figures 49 through 52 show various views of a two-part MDI adapter according to a further example.

Directional phrases used herein, such as, for example and without limitation, top, bottom, left, right, upper, lower, front, back, and derivatives thereof, relate to the orientation of the elements shown in the drawings and are not limiting upon the claims unless expressly recited therein.

As employed herein, the statement that two or more parts or components are "coupled" together shall mean that the parts are joined or operate together either directly or through one or more intermediate parts or components.

As employed herein, the statement that two or more parts or components "engage" one another shall mean that the parts exert a force against one another either directly or through one or more intermediate parts or components.

As employed herein, the term "number" shall mean one or an integer greater than one (i.e., a plurality).

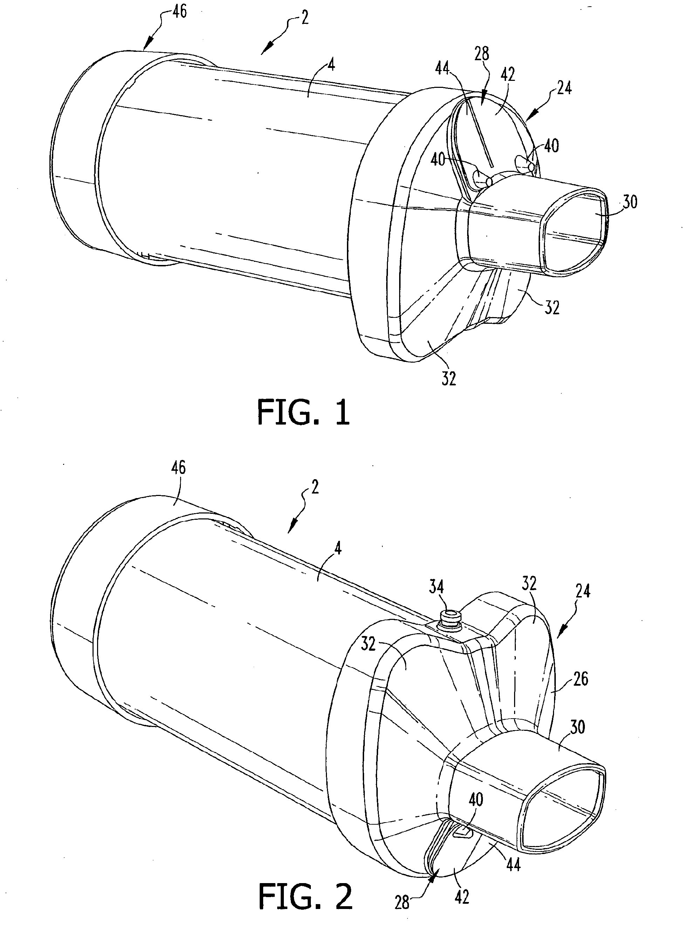

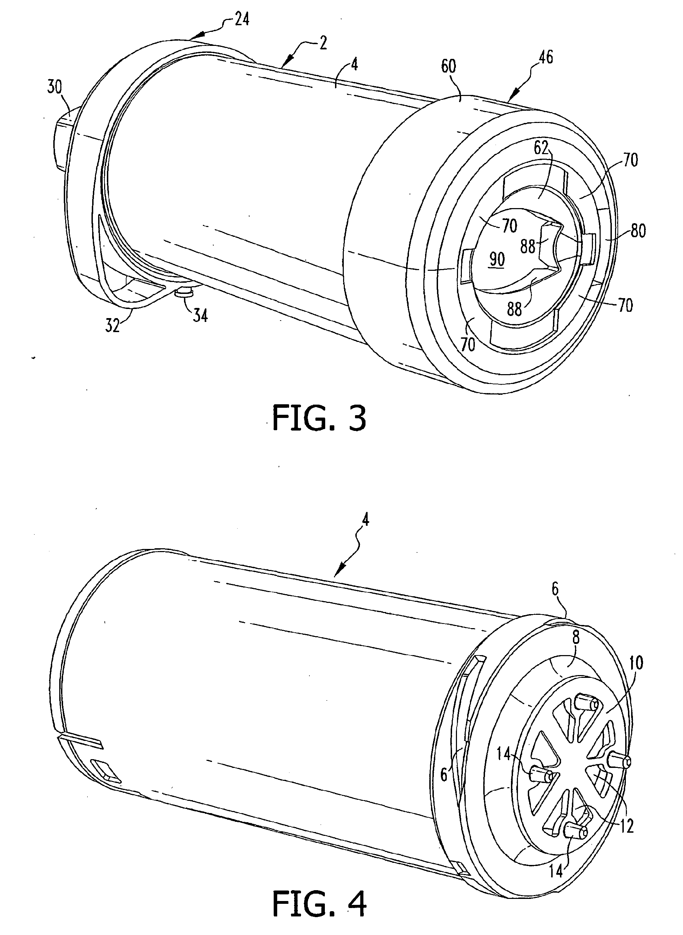

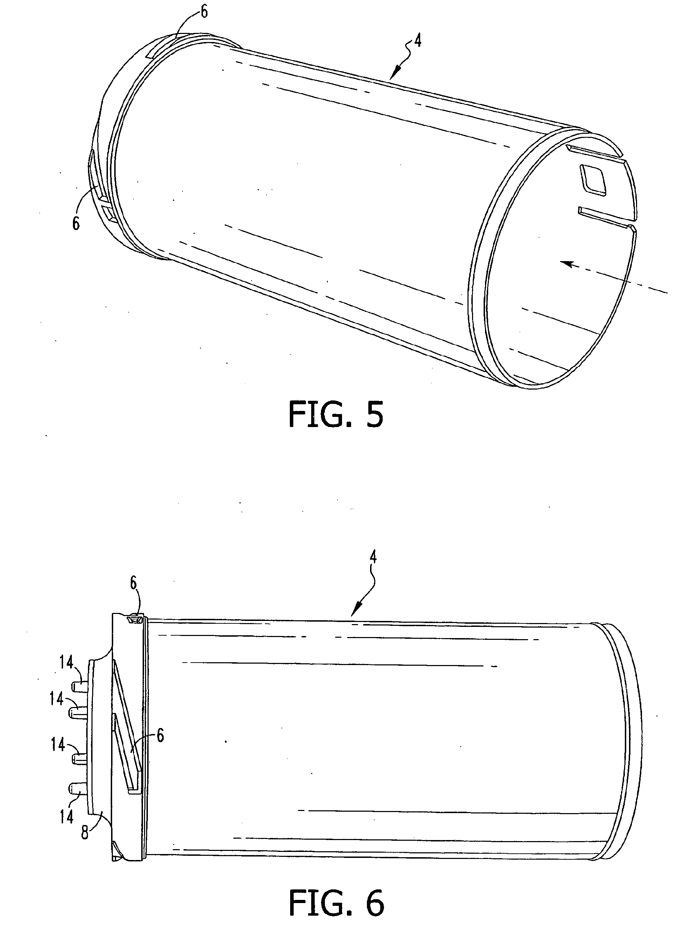

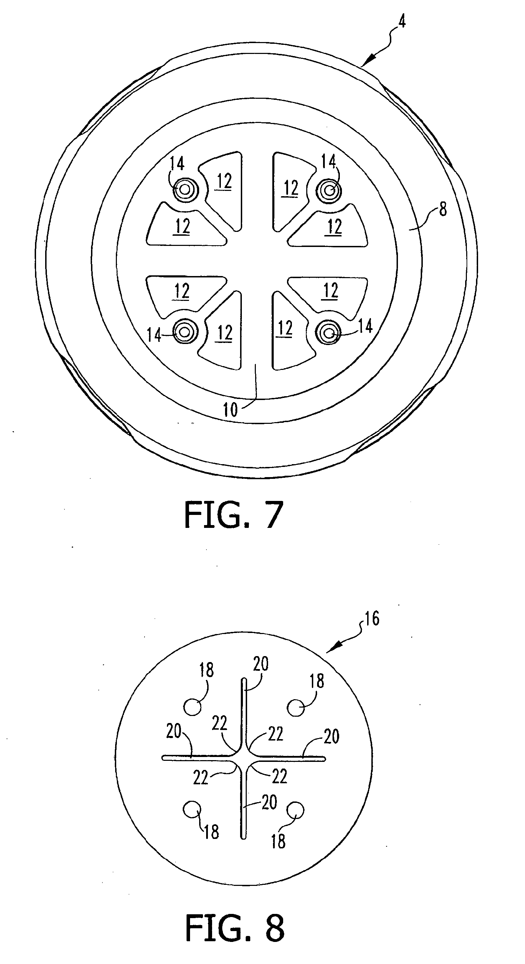

Figures 1, 2 and 3 are isometric views of a valved holding chamber 2 according to one embodiment of the present invention. The valved holding chamber 2 is structured to be used in connection with a metered dose inhaler (MDI) as described elsewhere herein. The valved holding chamber 2 includes a generally cylindrical main chamber housing 4 which is shown separately in Figures 4-7. In particular, Figures 4 and 5 are isometric views of the main chamber housing 4, Figure 6 is a side elevational view of the main chamber housing 4, and Figure 7 is a front elevational view of the main chamber housing 4. The front end of the main chamber housing 4 is, in the embodiment shown, formed with an external screw thread 6 and with an inwardly extending partition 8 having a top surface 10 defining a plurality of holes 12 which open into the interior of the main chamber housing 4. In addition, a plurality of pegs 14 each extends outwardly from the top surface 10. The main chamber housing 4 may be made of a static or an anti-static material.

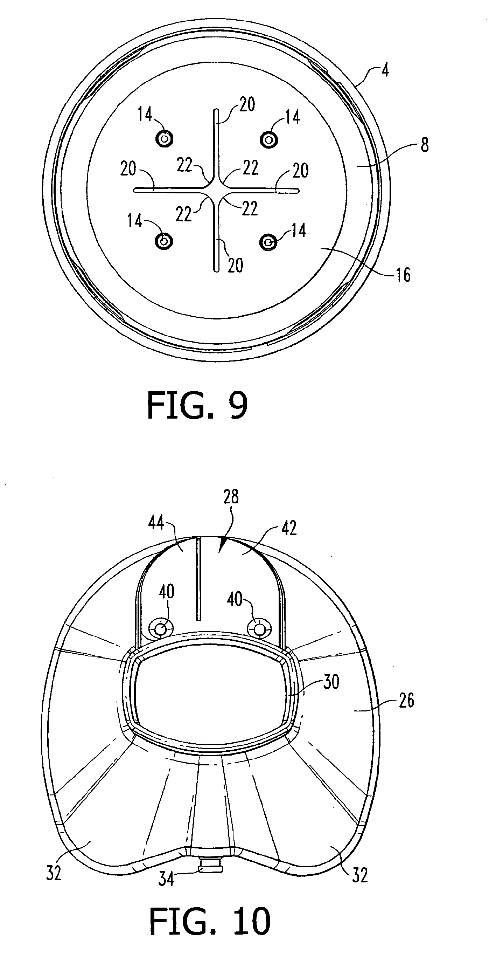

The valved holding chamber 2 includes a one-way inhalation valve which, in the present embodiment, is in the form of an elastomeric valve disk 16 having holes 18 as shown in Figure 8. As seen in Figure 9, the valve disk 16 is positioned on the top face 10 of the partition 8 of the main chamber housing 4 by inserting the pegs 14 through holes 18. The valve disk 16 also includes slits 20 which form flaps 22 that, when the valve disk 16 is positioned as shown in Figure 9, normally overlie and cover the holes 12 of the main chamber housing 4. As noted above, the valve disk 27 acts as a one-way valve which permits a fluid such as inhaled air to flow from within the interior of the main chamber housing 4 and out through the holes 12, and that prevents fluids such as exhaled gases from flowing from outside of the main chamber housing 4 and through the holes 12 into the interior of the main chamber housing 4. Specifically, as is known in the art, when a gas such as inhaled air flows in the direction of the arrow shown in Figure 5, the flaps 22 of the valve disk 16 will be caused to deflect outwardly, thereby uncovering the holes 12 and allowing the gas to pass therethrough. In contrast, when a gas such as an exhaled gas impinges upon the valve disk 16 in the opposite direction, the flaps 22 are forced against the top portion 10 of the partition 8, thereby sealingly covering the holes 12.

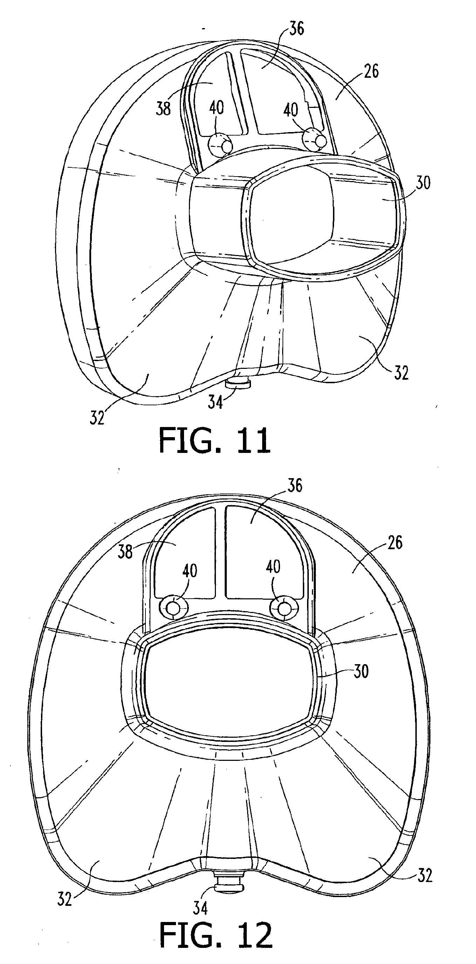

Referring again to Figures 1-3, a mouthpiece assembly 24 is, in the embodiment shown, threaded onto the screw thread 6 of the front end of the main chamber housing 4. Alternatively, the mouthpiece assembly 24 may be permanently attached to the main chamber housing 4. The mouthpiece assembly 24 is shown separately in Figure 10 and includes a main body portion 26 having a dual flap exhalation valve 28 operatively coupled thereto, the function of which is described elsewhere herein. The main body portion 26 of the mouthpiece assembly 24 is shown in Figures 11-13 and includes a mouthpiece 30 structured to be received within the lips of the patient during use of the valved holding chamber 2. Preferably, the portion of the mouthpiece 30 that is connected to the remainder of the main body portion 26 forms a 90 degree wall or shelf. As seen in Figures 11-13, the mouthpiece 30 has an oblong, tapered shape, but it should be understood that other shapes are possible, such as, without limitation, a circular shape. The main body portion 26 further includes first and second legs 32 which enable the valved holding chamber 2 to be rested and/or stored in a position as shown in Figure 1 without freely rolling from side-to-side (as would be the tendency with a cylindrically shaped body). This is advantageous for storage purposes as it prevents the valved holding chamber 2 from undesirably rolling in one direction or the other. The main body portion 26 further includes a peg 34 positioned between the legs 32 which is structured to hold a tether which is attached to a cap (not shown) which covers the mouthpiece 30 when not in use. The peg 34 is preferably recessed with respect to the bottoms of the legs 32 and is thereby protected from damage (for example, being broken off) when, for example, the valved holding chamber 2 is handled and/or dropped. The main body portion 26 further includes a first exhalation port 36 and a second exhalation port 38 through which gasses exhaled by a patient are able to pass as described in more detail below.

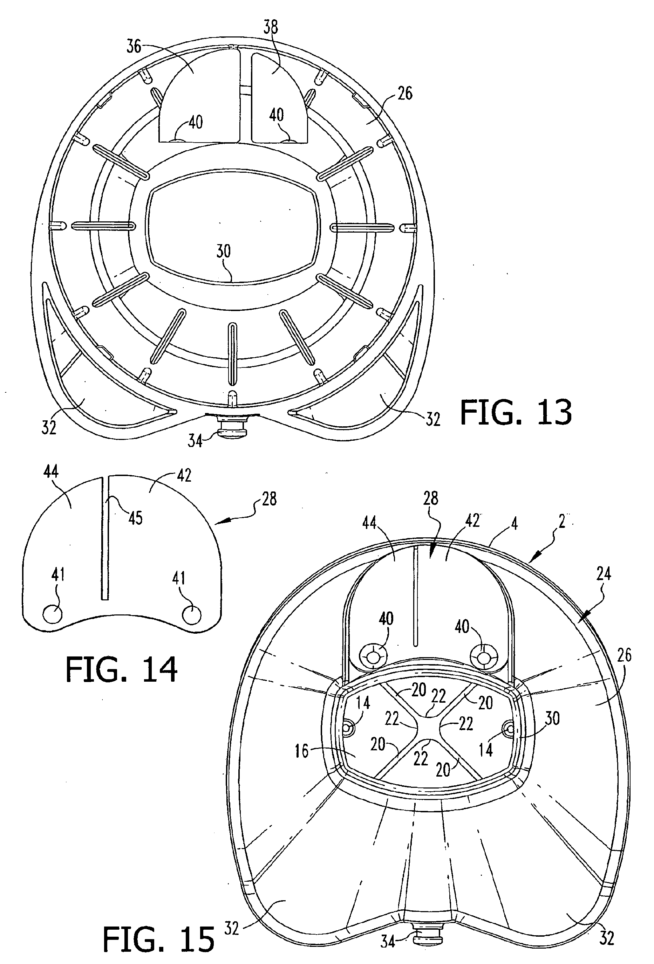

As seen in Figures 1, 2 and 10, the illustrated example of the valved holding chamber 2 includes an exhalation valve element in the form of a dual flap exhalation valve 28. A top plan view of the dual flap exhalation valve 28 is shown in Figure 14. The dual flap exhalation valve 28 is structured to be removeably or permanently attached to the main body portion 26 of the mouthpiece assembly 24 by being positioned over the exhalation ports 36 and 38 in a manner in which pegs 40 provided as part of the main body portion 26 are received through holes 41 provided in the dual flap exhalation valve 28. The dual flap exhalation valve 28 is preferably made of a flexible material such as, without limitation, silicone, rubber, a thermoplastic elastomer (TPE), Mylar, plastic, paper or foam, among other materials.

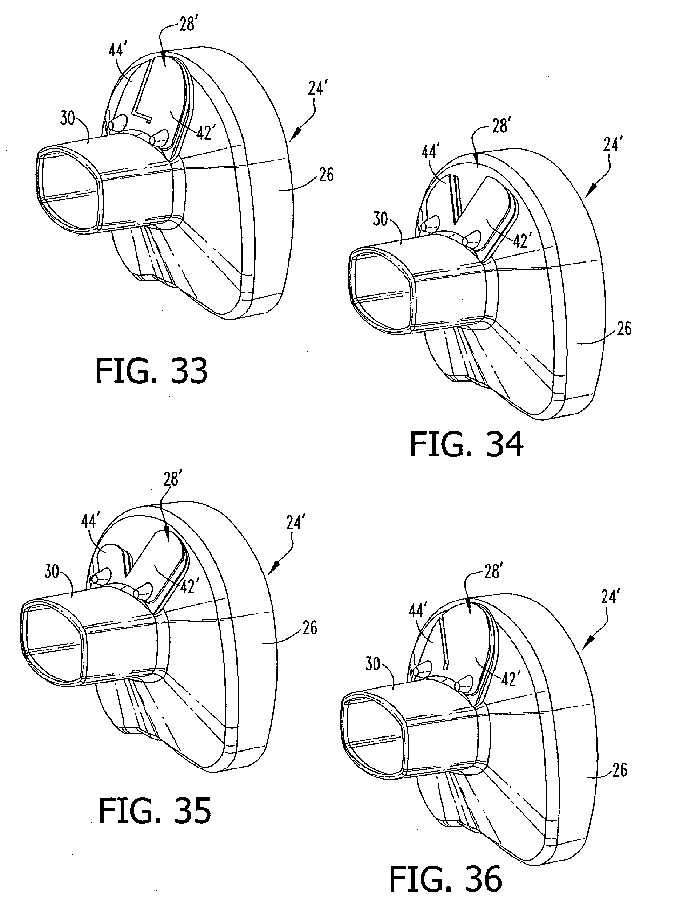

The preferred dual flap exhalation valve 28 includes a first flap portion 42 and a second flap portion 44 separated by a slit. As used herein, the term "flap" or "flap portion" shall mean any element that is attached to a surface or other element at one end and hangs lose as is free to move at the other end. The first flap portion 42 has a first cross-sectional area that is greater than a second cross-sectional area of the second flap portion 44. In addition, the cross-sectional area of the first exhalation port 36 is greater than the cross-sectional area of the second exhalation port 38. These characteristics cause the first flap portion 42 to have a lower resistance to gas flow than the second flap portion 44. As a result, at low flow rates (e.g., below some threshold level), the first flap portion 42 will be caused to move more significantly than the second flap portion 44, which, depending upon the level of flow rate, may not move at all. At high flow rates (e.g., above some threshold level), both the first flap portion 42 and the second flap portion 44 will be caused to move significantly in a similar manner and to a similar extent. In each case, the flaps 42, 44 will be caused to move generally away from the corresponding exhalation port 36, 38. This difference in response to various flow rates will occur as long as the cross-sectional areas of the exhalation ports 36 and 38 are different from one another. As indicated above, the cross-sectional areas of the flaps 42 and 44 are preferably also made to be different than one another, although it is possible to use flaps 42, 44 having similar cross-sectional areas with exhalation ports 36 and 38 having cross-sectional areas that are different from one another. Other example examples of suitable exhalation valve elements that function in this manner are described elsewhere herein (Figures 33-39).

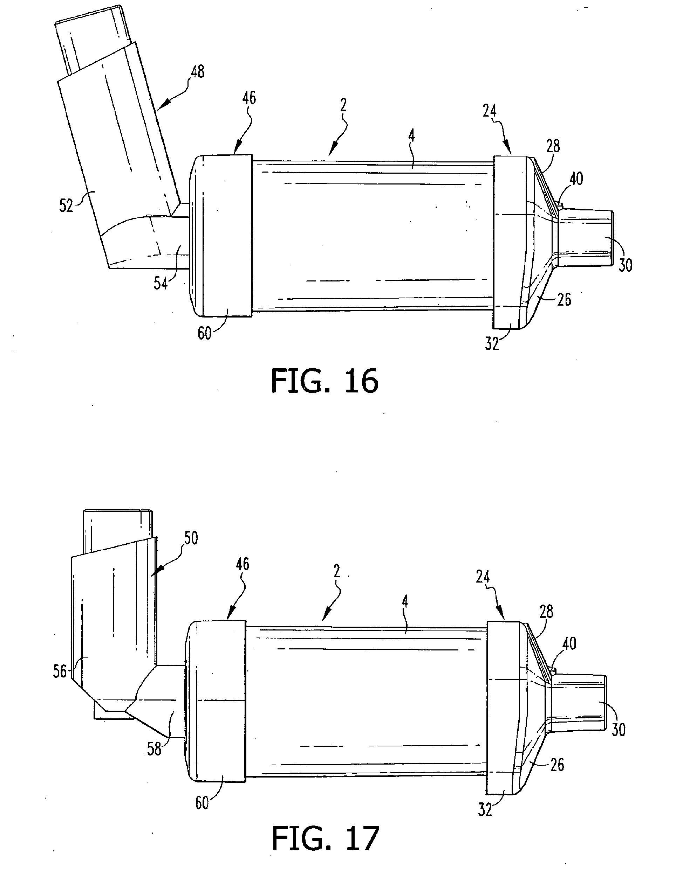

As is known in the art, when the valved holding chamber 2 is used by a patient, the patient inserts the mouthpiece 30 into his or her mouth and exhales in order to at least partially empty gas from the patient's lungs. The exhaled gasses are, through operation of the dual flap exhalation valve 28, allowed to flow from within the mouthpiece assembly 24 to the ambient atmosphere through one or both of the exhalation ports 36 and 38 and, as described elsewhere herein, such gasses are not, as a result of the operation of the valve disk 16, permitted to flow into the interior of the main chamber housing 4. Following exhalation, the patient actuates the MDI that is inserted within the valved holding chamber 2 (see Figures 16 and 17 described elsewhere herein) in order to cause a dose of medication to be sprayed within the main chamber housing 4, and thereafter begins inhaling in order to deposit the medication (mixed with air in the main chamber housing 4) within the patient's lungs. During inhalation, the first flap portion 42 and the second flap portion 44 will move toward the exhalation ports 36 and 38 to thereby create a seal so that no medicine is caused to escape through the exhalation ports 36 and 38.

According to an aspect of the invention, the dual flap exhalation valve 28 is used to signal to the patient and/or a caregiver that exhalation is occurring and when exhalation has stopped. In particular, at the beginning of exhalation, for adults, flow rates will typically be high, and therefore both the first flap portion 42 and the second flap portion 44 will be caused to move significantly (away from the respective exhalation port 36, 38). Towards the end of exhalation, flow rates will typically become lower, and, depending upon the flow rate level, typically only the first flap portion 42 will continue to move (i.e., when flow rates drop below the threshold level at which the second flap portion 44 is caused to move). When both the first flap portion 42 and a second flap portion 44 stop moving, that is a visual signal to the patient and/or the caregiver that exhalation has stopped and that the user can then actuate the MDI and begin inhalation. For children, exhalation flow rates will typically be lower, and, in most cases, only the first flap portion 42 will be caused to move during exhalation. When the first flap portion 42 stops moving, that is a visual signal to the child patient and/or the caregiver that exhalation has stopped and that the MDI may thereafter be actuated and inhalation may begin.

As is known in the art, after the MDI has been actuated and a patient has inhaled so as to cause the medicine to be received within the patient's lungs, it is important for the patient to hold their breath and not exhale for a certain period of time (e.g., four or five seconds) so that the medicine can be deposited within the lungs of the patient. If a patient exhales too soon after inhalation, the medicine, rather than being deposited within lungs, will simply be exhaled and therefore not absorbed by the patient. The dual flap exhalation valve 42 therefore also acts as a visual guide for a patient to make sure they are not exhaling but rather are holding their breath after inhalation has occurred. In this respect, the patient will watch the dual flap exhalation value 28 to make sure that neither the first flap portion 42 nor the second flap portion 44 moves for the predetermined desirable period of time. Furthermore, the movement of the first flap portion 42 and a second flap portion 44 of the dual flap exhalation valve 28 is a visual indicator which enables a caregiver to more easily count breaths so as to assist in determining the end of a particular treatment. Finally, if the valved holding chamber 2 is used with a mask, the dual flap exhalation valve 28 may be used to indicate that a proper mask seal has been obtained.

The dual flap exhalation valve 28 shown in Figure 14 is just one particular embodiment that may be used in the valved holding chamber 2. It should be understood, however, that additional embodiments of suitable exhalation valve elements may also be used. For example, a dual flap exhalation valve may be created wherein one flap will have a greater resistance to gas flow than the other flap due to a difference in thickness of the material of the particular flap (the thicker the material, the greater the resistance to flow), due to a difference in durometer of the material of the particular flap (the higher the durometer, the greater the resistance to flow), or due to difference in the length of the hinge portions which connect the flaps to the main body portion 26 of the mouth piece assembly 24 (the longer the hinge, the greater the resistance to flow).

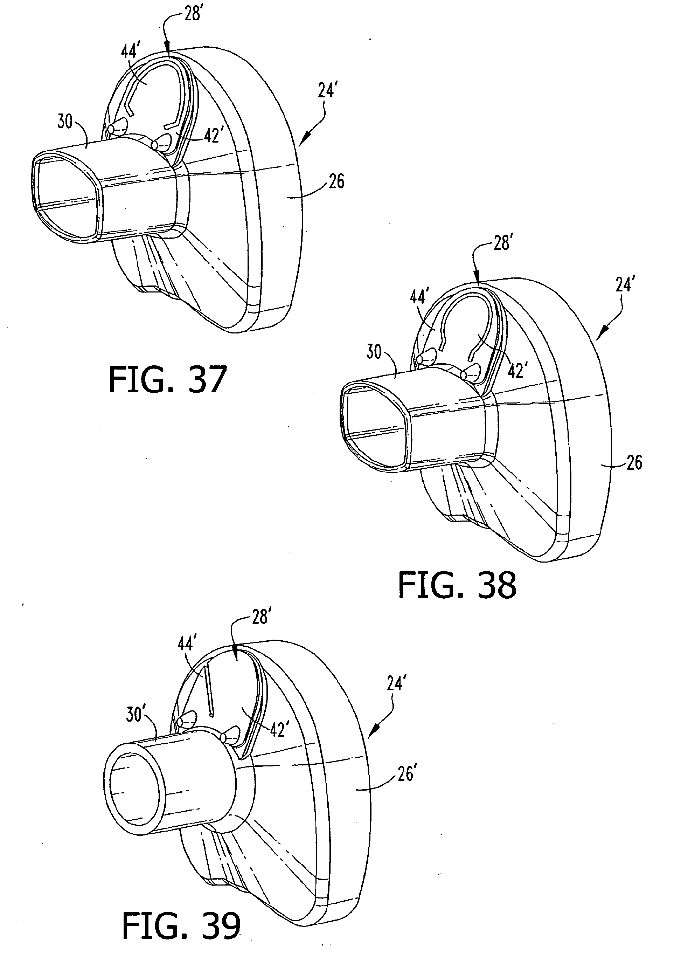

In addition, a number of other exhalation valve element embodiments are possible, with the important feature being that the exhalation valve element includes first and second portions that move to a different extent at lower flow rates, i.e., below, for example, some predetermined threshold level (one portion may not move at all at certain of such lower flow rates). At higher flow rates (above, for example, that predetermined threshold level), the first and second portions may move in a similar manner. As noted above, the different resistances to flow rates may be dependent upon characteristics including size, thickness and durometer. A number of such alternative embodiments are shown in Figures 33 to 39. In those Figures, each alternative dual flap exhalation valve is labeled with the reference numeral 28', and each alternative mouthpiece assembly of which they are a part is labeled 24'. Again, each of the exhalation valve elements that is shown have in common the feature that a first portion thereof (labeled 42') moves more significantly in response to lower flow rates than a second portion thereof (labeled 44'). Preferably, each first portion 42' and each second portion 44' covers an exhalation port having a cross sectional area that is similar in size to the corresponding portion 42', 44'. Furthermore, the two portions may be connected to one another to form a unitary valve assembly component (as is the case with the preferred dual flap exhalation valve 28), or may be made separate pieces which together form the valve assembly. In addition, Figure 39 shows a main body portion 26' of the mouthpiece assembly 24' that has a round (as opposed to oblong) mouthpiece 30'.

The dual flap exhalation valve 28 is shown in the Figures as being removable from the mouthpiece assembly 24. This may be advantageous as it allows the dual flap exhalation valve 28 to be removed for cleaning and/or if it becomes damaged. However, it should be understood that the dual flap exhalation valve 28 may instead be permanently affixed to the main body portion 26 of the mouthpiece assembly 24.

In addition, in the preferred embodiment of the dual flap exhalation valve 28 shown in Figure 14, one advantage thereof is that because it is in the form of two flaps 42 and 44, the amount of movement as a result of patient exhalation will be significant and therefore will be able to be viewed from a distance by, for example, a caregiver. As a result, the preferred embodiment shown in Figure 14 provides a good visual indicator function.

As described elsewhere herein, one function of the dual flap exhalation valve 28 is to allow the caregiver to count the number of breaths that the patient has taken, especially in children. Traditional flap valves are designed to give low resistance, which is achieved by having a large flap which opens a small distance (1-2 mm). This small movement is not obvious to the user. As described elsewhere herein, the two flap design (e.g., the dual flap exhalation valve 28) is preferably configured to achieve a large degree of movement in a first flap portion at a low flow rate which is easily observed and counted, while a second flap portion provides low resistance only at high flows. In one particular, non-limiting embodiment, a two flap design may be achieved by having the first flap portion have the following characteristics: (i) low resistance at low flows (<1cm Wg) due to, for example, the thickness or durometer of the material used to make the first flap portion, (ii) a small cross-sectional area, and (iii) a cowl provided around the port to prevent air from escaping as it opens until there is significant movement of the first flap portion; and by having the second flap portion have the following characteristics: (i) a certain fixed resistance to opening (e.g. >1cm Wg required to open) to ensure that the first flap portion opens first due to, for example, the thickness or durometer of the material used to make the second flap portion, , (ii) a large area cross-sectional area, and (iii) no cowl.

Figure 15 is a front view of the valved holding chamber 2 which shows the mouthpiece assembly 24 (including the dual flap exhalation valve 28) attached to the main chamber housing 4 by threading the mouthpiece assembly 24 onto the main chamber housing 4 using the screw threads 6. The valve disk 16 can be seen through the mouthpiece 30 in this view.

According to a further aspect of the illustrated example, the valved holding chamber 2 further includes a two-part MDI adapter 46 which is structured to be removeably attached to the end of the main chamber housing 4 that is opposite the mouthpiece assembly 24. The MDI adapter 46 is structured to receive and hold an MDI such as MDI 48 shown in Figure 16 or an MDI 50 shown in Figure 17. In particular, the MDI adapter 46 is structured to receive and hold MDI's having canister holders having outlets of differing shapes. For example, the MDI 46 is able to securely hold the MDI 48 which has a canister holder 52 having an outlet 54 that has an oblong, semi-oval shape. In addition, the MDI adapter 48 is also able to securely hold the MDI 50 which has a canister holder 56 having an outlet 58 that has a round shape.

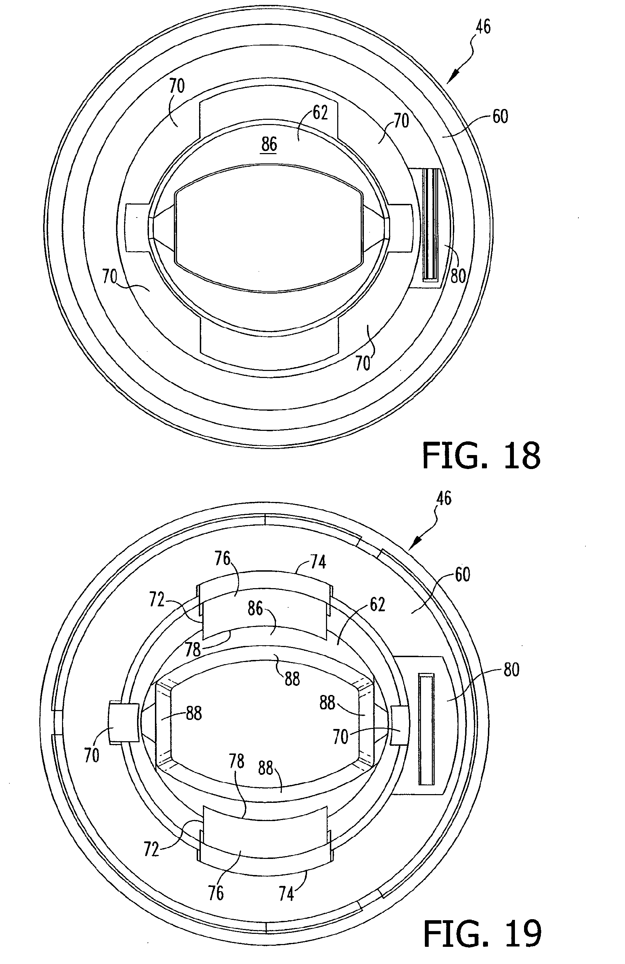

Figures 18 and 19 are front and rear views, respectively, of the two-part MDI adapter 46. The two-part MDI adapter 46 includes a rigid end cap 60 made of, for example, a hard plastic or some other suitable rigid material that is structured to be selectively attachable to the end of the main chamber housing 4. The two-part MDI adapter 46 further includes a flexible inner portion 62 made of a flexible material, such as, without limitation, silicone, rubber, TPE, or foam, among other suitable materials. The inner portion 62 is structured to be received in and held by the end cap 60 and may be made to be removable so that it can be cleaned and/or replaced if damaged, or, alternatively, may be permanently affixed to the end cap 60 by a process such as an over-molding process.

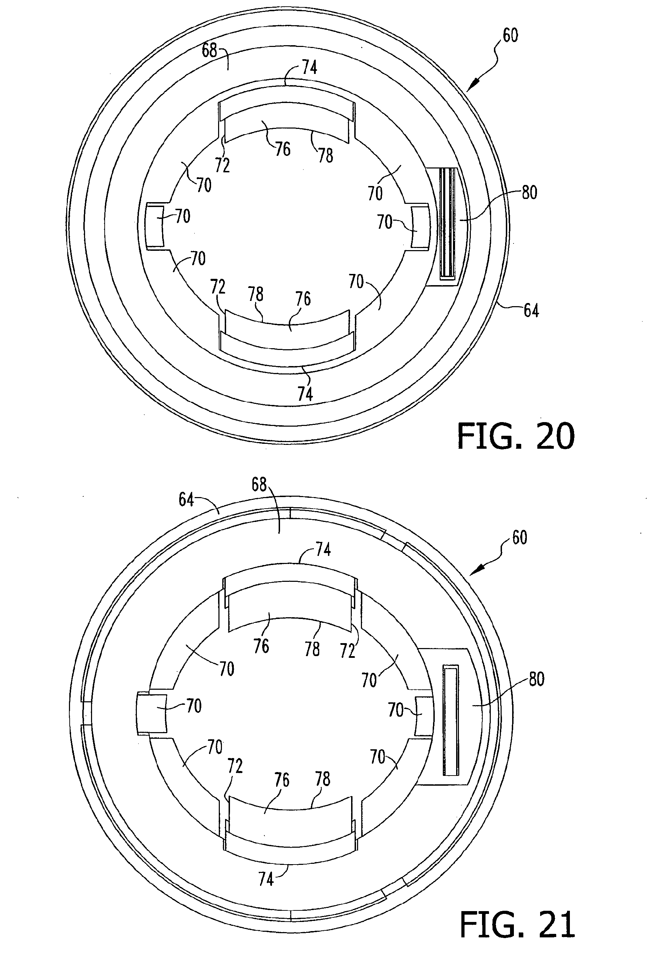

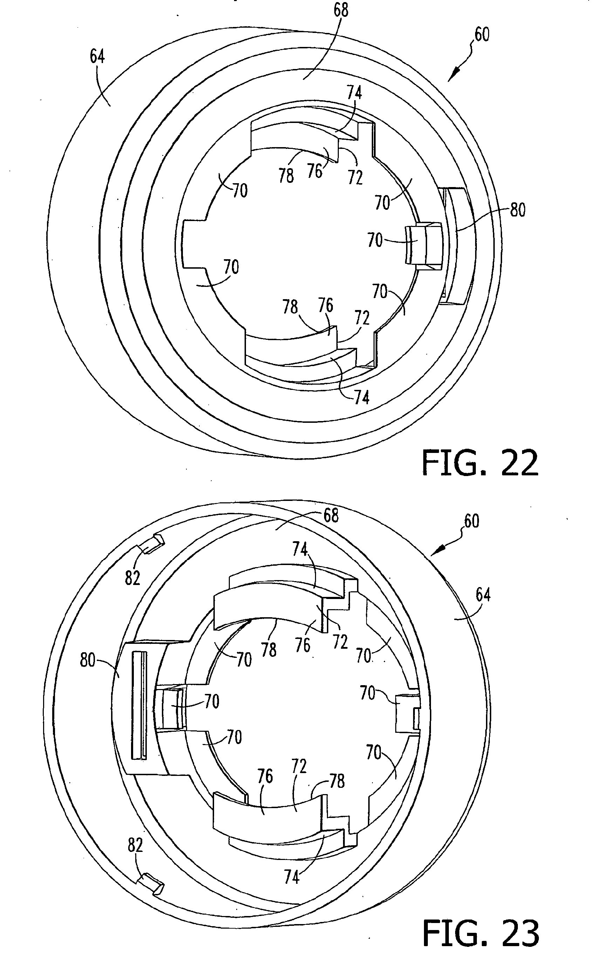

Figure 20 is a front elevational view and Figure 21 is a rear elevational view of the end cap 60. Figures 22 and 23 are isometric views of the end cap 60. The end cap 60 includes an outer wall 64 connected to an end wall 68. A plurality of flanges 70 extend from the end wall 68 and are structured to receive and hold the inner portion 62 therebetween as shown in, for example, Figures 18 and 19. Furthermore, first and second support members 72 extend from the inner wall 68 and include a first portion 74 that extends generally parallel to the longitudinal axis of the end cap 60 and a second portion 76 that extends generally perpendicular to the longitudinal axis of the end cap 60. Furthermore, as seen in Figures 20-23, the second portion 76 has an arcuate outer face 78. The purpose and functionality of the support members 72 is described below. In addition, the end cap 60 has an airflow actuated noisemaker 80 integrally formed therein. The noisemaker 80 may be, for example, a whistle as shown in Figures 20-23 or, alternatively, a device including a sound reed which is caused to vibrate by air flowing thereover. The noisemaker 80 in the example shown in the Figures is a high airflow indicator that is structured to generate a noise when the air flowing through the main chamber housing 4 as a result of patient inhalation exceeds some predetermine level. Thus, the noisemaker 80 provides a cautionary indication to the patient that the patient is inhaling too quickly and should slow down. Finally, the end cap 60 includes flanges 82 for connecting the end cap 60 to the main chamber housing 4.

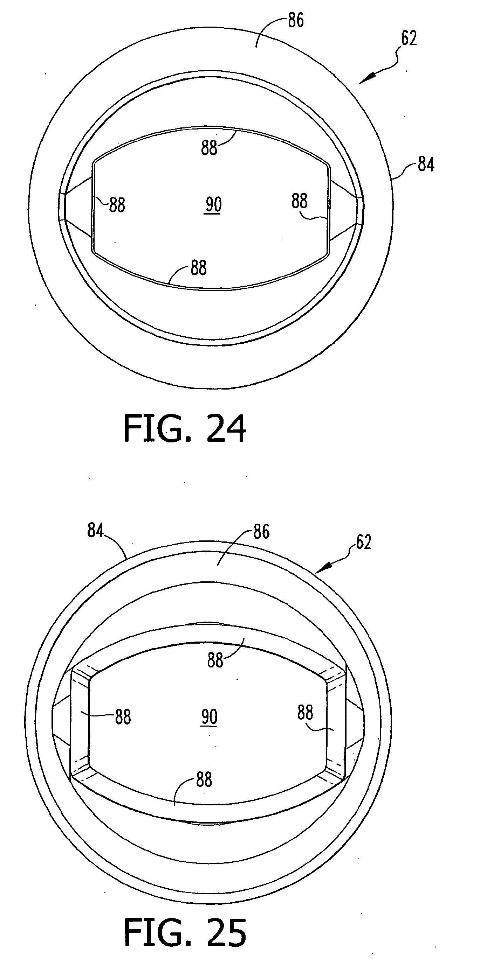

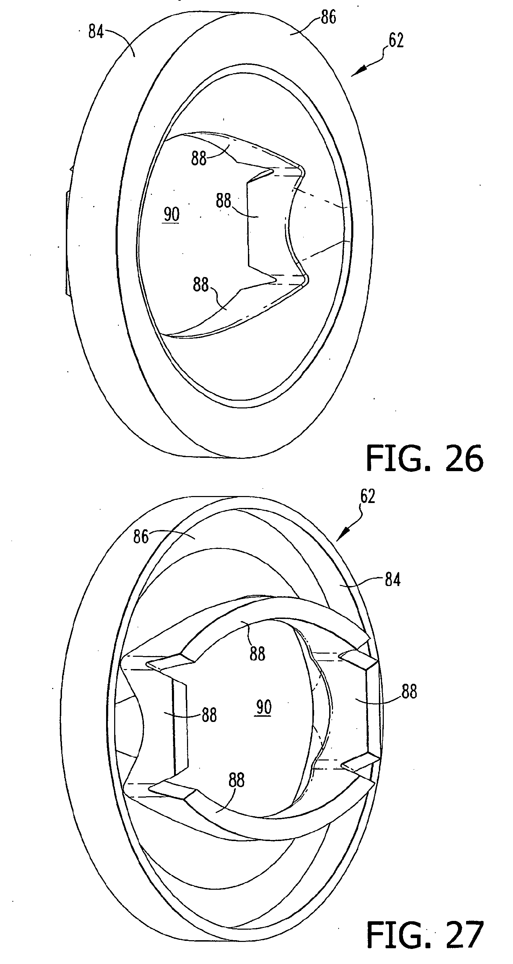

Figures 20 and 25 are front elevational and rear elevational views, respectively, of the flexible inner portion 62. Figures 26 and 27 are isometric views of the flexible inner portion 62. The flexible inner portion 62 includes an outer wall 84 connected to an end wall 86. Extending from the end wall 86 in a direction that is substantially parallel to longitudinal axis of the inner portion 62 are upstanding inner walls 88 which define an aperture 90. The aperture 90 is provided to receive the outlet of an MDI, such as the outlet 54 of the MDI 48 or the outlet 58 of the MDI 50.

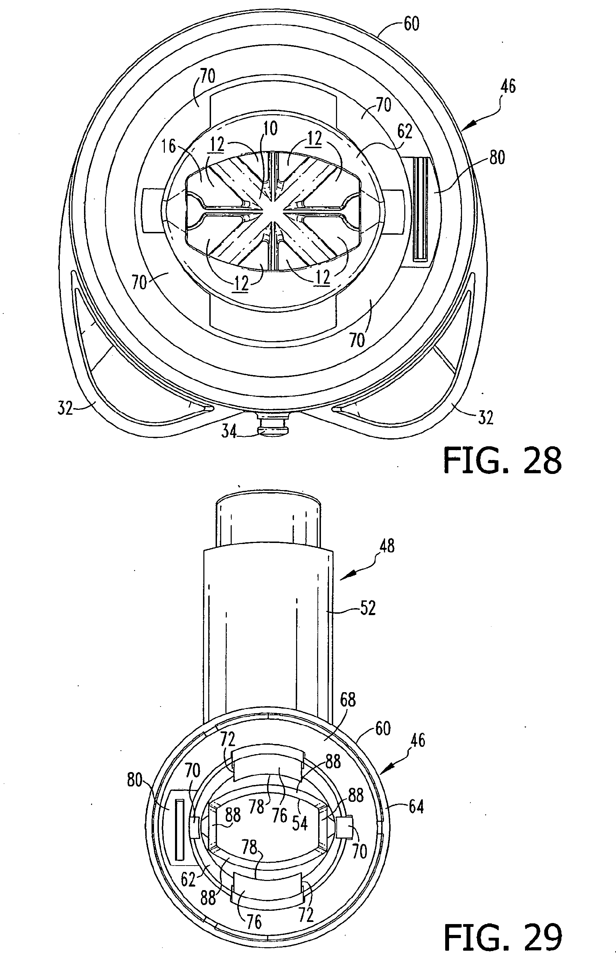

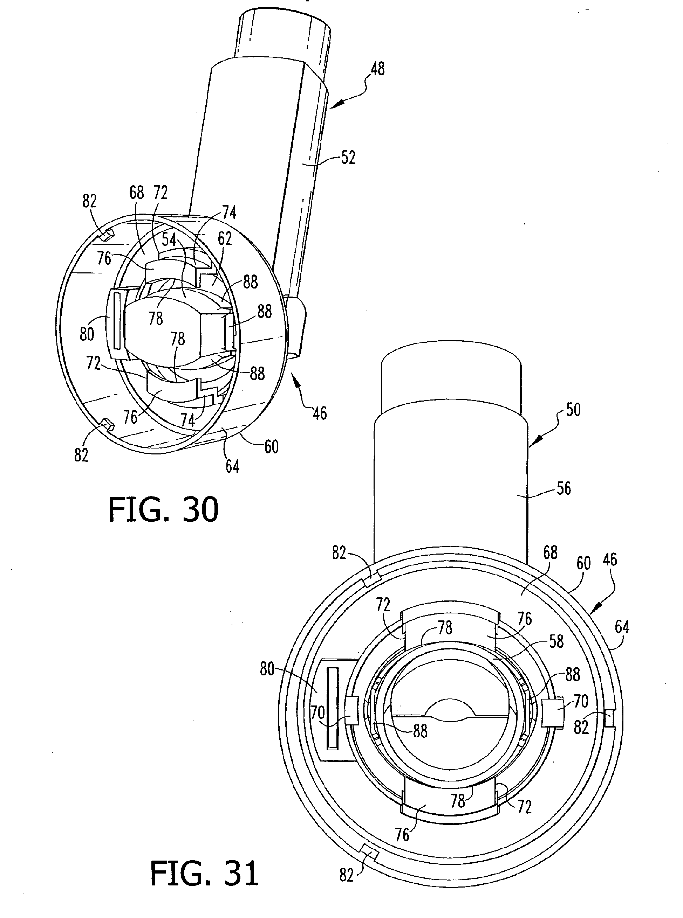

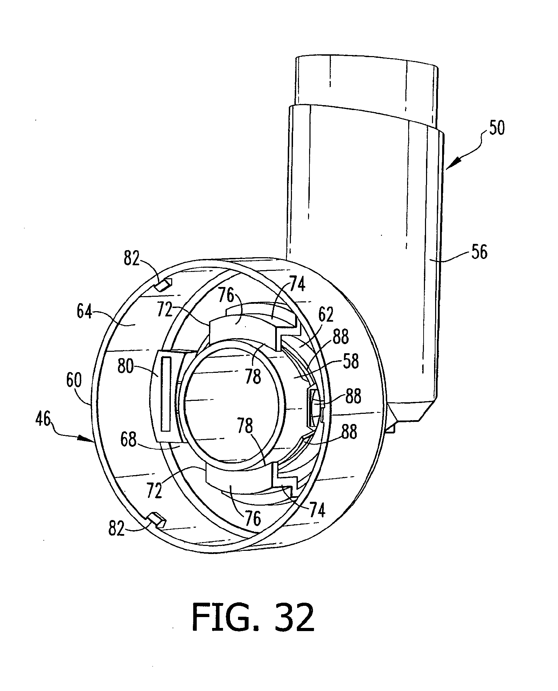

As noted elsewhere herein, the MDI adapter 46 is flexible and is structured to receive and securely hold MDI's having outlets of differing shapes. Figure 28 is a rear view of the valved holding chamber 2 showing the end cap 46 attached thereto ready to receive an MDI such as the MDI 48 or the MDI 50. Figures 29 and 30 are front elevational and isometric views, respectively, of the MDI adapter 46 holding the MDI 48 having an outlet 54 that has an oblong, generally oval shape. As seen in Figures 29 and 30, the outlet 54 is received in the aperture 90 and is held around the outer periphery thereof by the inner walls 88 of the inner portion 62. Figures 31 and 32 are front elevational and isometric views, respectively, of the MDI adapter 46 securely holding the MDI 50 having an outlet 58 that has a generally round shape. As seen in Figures 31 and 32, the outlet 58 is received through the aperture 90 of the inner portion 62. The outer periphery of the outlet 58 is surrounded by and supported by the inner walls 88 of the inner portion 62. However, in contrast to Figures 29 and 30, the outlet portion 58 also engages and is supported by the arcuate surfaces 78 of the support member 72. Thus, as illustrated in Figures 29-32, the MDI adapter 46 is able to securely hold both the MDI 48 having an oblong outlet 54 and the MDI 50 having a round outlet 58. In the case of the MDI 50 having a round outlet 58, the rigid support members 72 support and assist in positioning the MDI 50 and reduce tilting of the round outlet 58 during actuation of the MDI 50 (such titling is common when round MDIs are supported by prior art adapters made entirely of a flexible material (e.g., a high durometer flexible material) due to a lack of sufficient support; such tilting, if present, can result in the medication being deposited in the walls of the associated chamber).

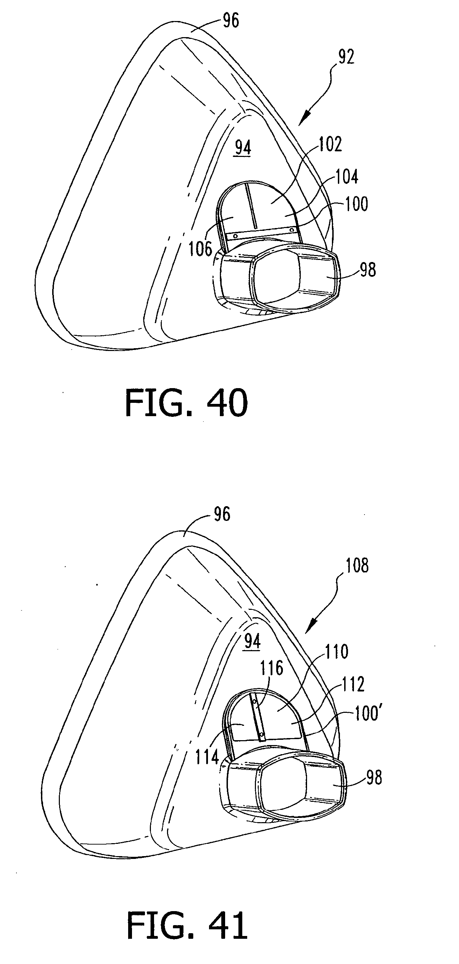

As described elsewhere herein, a mask may be employed with a valved holding chamber so that the patient can more freely breath through his or her mouth to receive the medication. Figure 40 is an isometric view of a mask 92 that may be used in connection with a valved holding chamber, such as, without limitation, the valved holding chamber 2, or some other type of respiratory drug delivery apparatus. The mask 92 includes a main body 94 having a seal element 96 attached thereto that is structured to surround the mouth of the patient. The main body 94 is preferably, although not necessarily, a generally rigid shell, whereas the seal element 96 is a flexible, resilient unitary member made of, for example, an elastomer such as plastic, rubber, silicone, vinyl or foam. The main body 94 includes a mouthpiece adapter 98 which is structured to receive and hold the mouthpiece of the valved holding chamber with which the mask 92 is used. The main body 94 also includes a valve assembly 100 that includes an exhalation valve element preferably in the form of a dual flap exhalation valve 102 that is similar in structure and function to the dual flap exhalation valve 28. The dual flap exhalation valve 102 has first and second flap portions 104, 106 having differing cross-sectional areas which cover respective exhalation ports having differing cross-sectional areas as described elsewhere herein. At low flow rates (e.g., below some threshold level), the first flap portion 104 will be caused to move more significantly than the second flap portion 106, which, depending upon the level of flow rate, may not move at all. At high flow rates (e.g., above some threshold level), both the first flap portion 104 and the second flap portion 106 will be caused to move significantly in a similar manner and to a similar extent. Figure 41 is an isometric view of an alternative example of a mask 108 that may be used in connection with a valved holding chamber, such as, without limitation, the valved holding chamber 2. The mask 108 is similar to the mask 92, except that it includes a valve assembly 100' having a dual flap exhalation valve 110 that is different in structure than (but similar in functionality to) the dual flap exhalation valve 102. In particular, the dual flap exhalation valve 110 includes first and second flap portions 112, 114 having differing cross-sectional areas which cover respective exhalation ports having differing cross-sectional areas as described elsewhere herein and which are each fixed at a central portion 166 so that they each move relative to the central portion 116. It should be understood that Figures 40 and 41 are meant to be exemplary, and that other exhalation valve element embodiments as described elsewhere herein (e.g., Figure 33-39) may also be used with a mask in the manner shown in Figures 40 and 41.

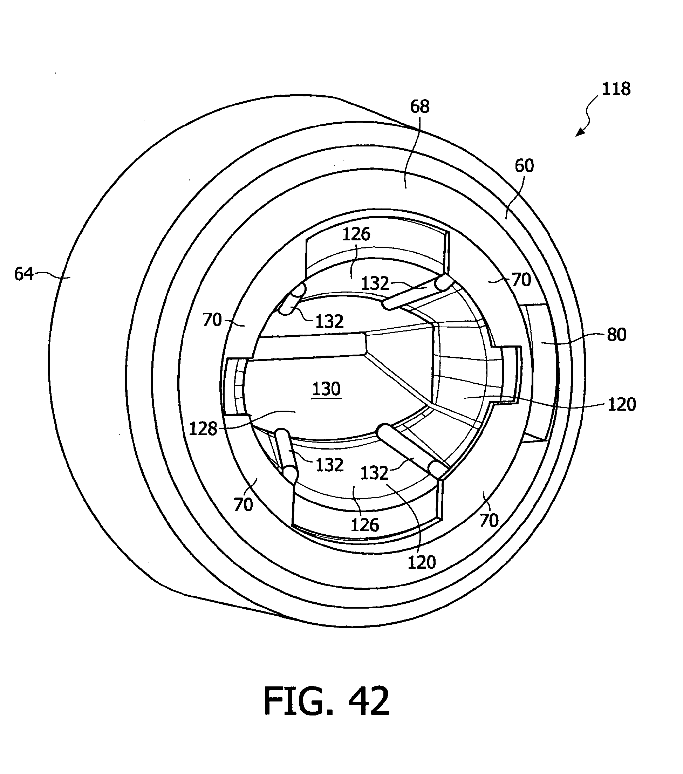

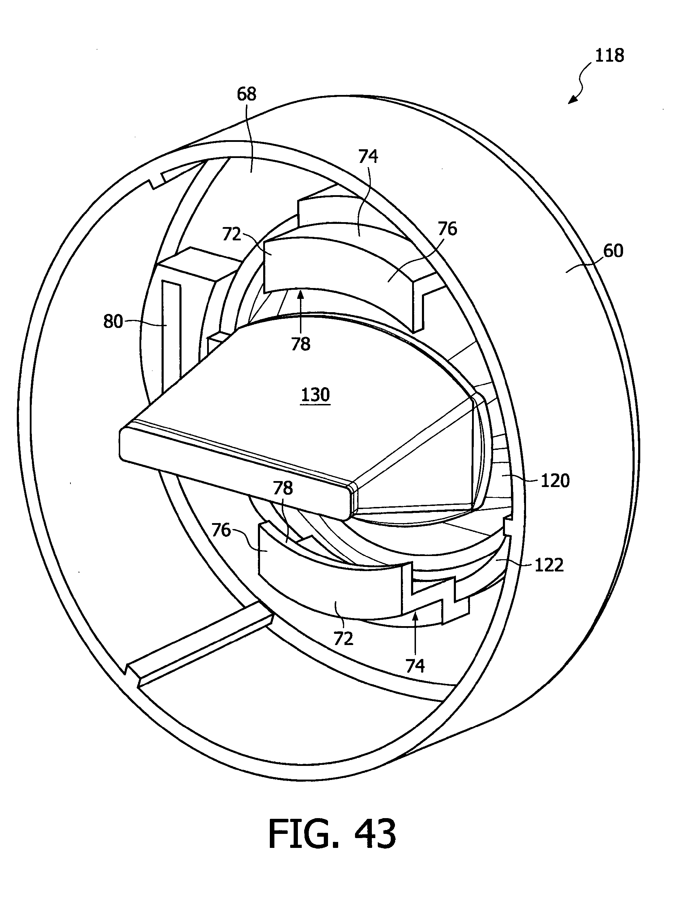

Figure 42 is a front isometric view and Figure 43 is a rear isometric view of a two-part MDI adapter 118 according to an example which is structured to be removeably attached to the end of the main chamber housing 4 that is opposite the mouthpiece assembly 24. The MDI adapter 118 is structured to receive and hold an MDI such as MDI 48 shown in Figure 16 or MDI 50 shown in Figure 17. The MDI adapter 118 includes the rigid end cap 60 described elsewhere herein (e.g., in connection with the MDI adapter 46 described elsewhere herein) made of, for example, a hard plastic or some other suitable rigid material that is structured to be selectively attachable to the end of the main chamber housing 4. As described elsewhere herein, the end cap 60 includes the rigid support members 72 having the arcuate surfaces 78 for supporting MDIs having round outlets such as MDI 50. The MDI adapter 118 further includes an alternative flexible inner portion 120 made of a flexible material, such as, without limitation, silicone, rubber, TPE, or foam, among other suitable materials. The inner portion 120 is structured to be received in and held by the end cap 60 and may be made to be removable so that it can be cleaned and/or replaced if damaged, or, alternatively, may be permanently affixed to the end cap 60 by a process such as an over-molding process.

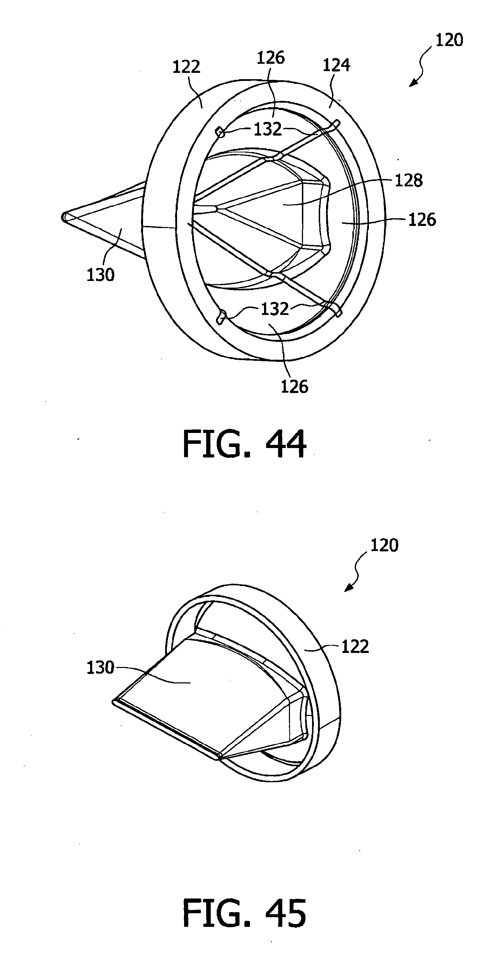

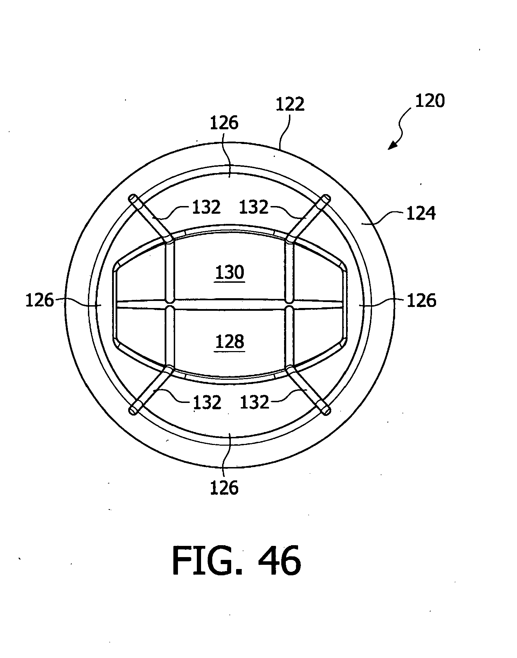

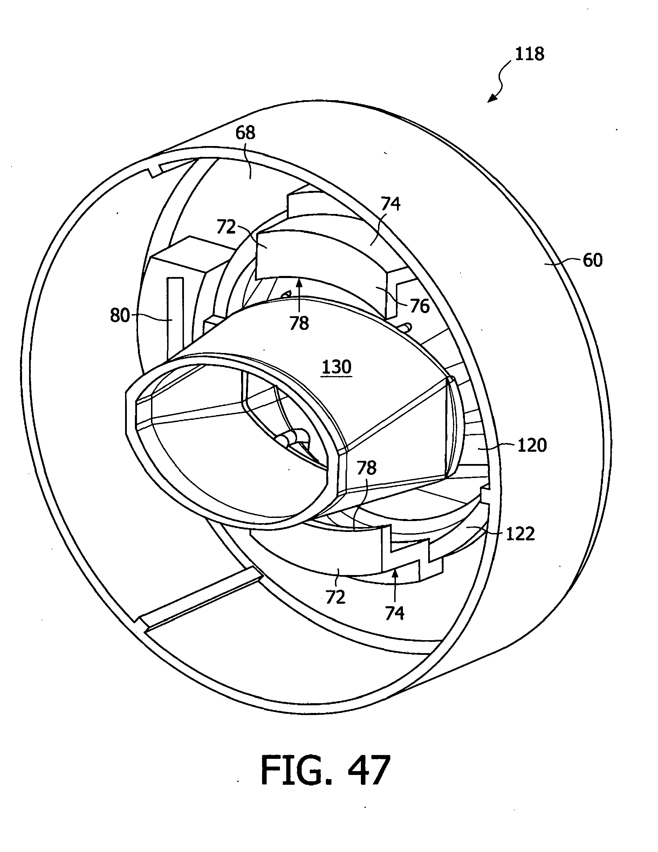

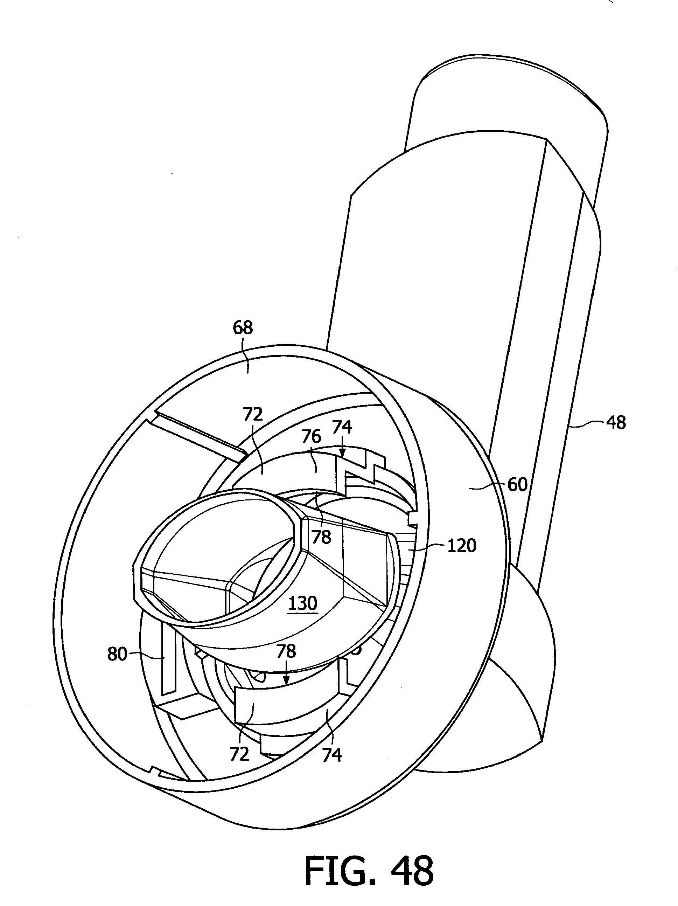

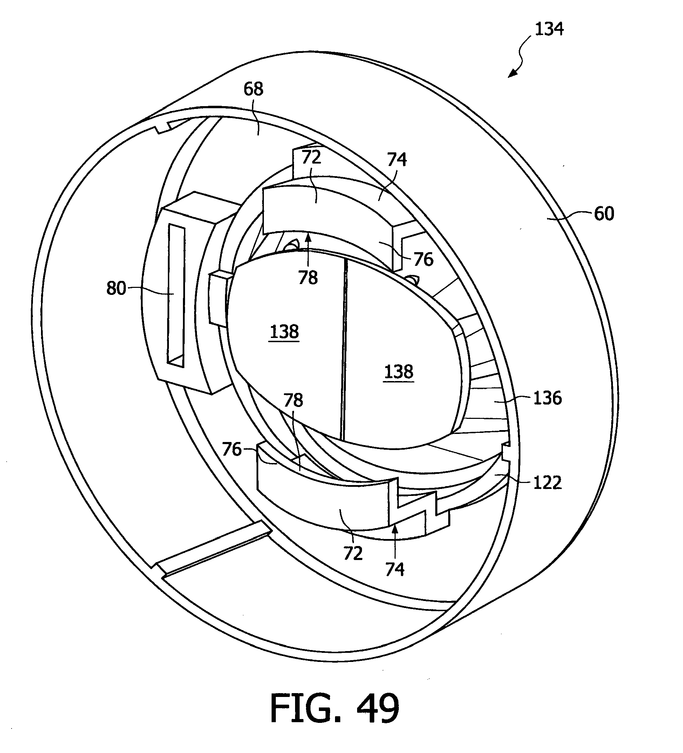

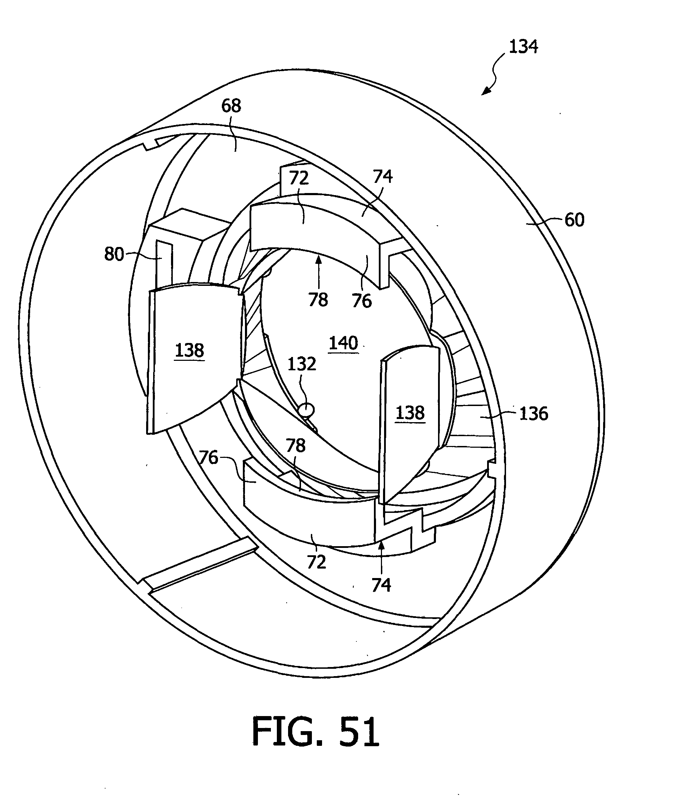

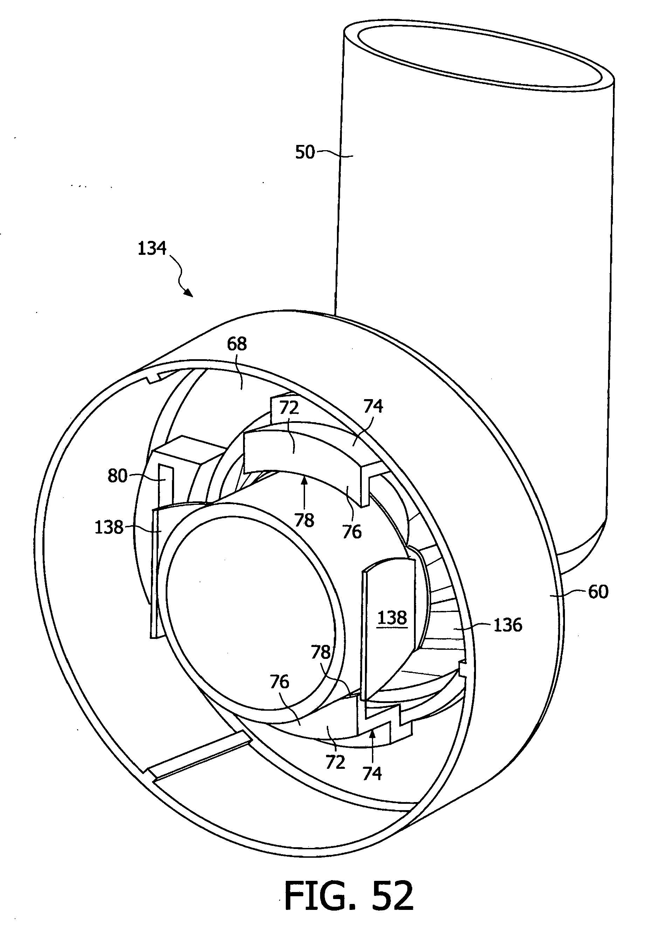

Figures 44 and 45 are front and rear isometric views, respectively, and Figure 46 is a front elevational view of the flexible inner portion 120 according to this example. The flexible inner portion 120 includes an outer wall 122 connected to an end wall 124. Extending from the end wall 124 downwardly and toward the rear of the inner portion 120 are inner walls 126, which at the ends thereof define an aperture 128. The aperture 128 is provided to receive the outlet of an MDI, such as the outlet 54 of the MDI 48 or the outlet 58 of the MDI 50. In addition, the inner portion 120 further includes an automatic protective closure mechanism, which in the example shown is in the form of a duckbill type valve 130, that is structured to (i) open and receive and support/hold the outlet of an MDI when it is inserted into the aperture 128, and (ii) automatically close when the MDI is removed from the MDI adapter 118, thereby closing off the main chamber housing 4 and preventing foreign objects from entering the main chamber housing 4. Figure 43 shows the duckbill type valve 130 in its closed condition, Figure 47 shows the duckbill type valve 130 in its open condition (without an MDI), and Figure 48 shows the duckbill type valve 130 in its open condition with the MDI 48 inserted in the MDI adapter 118 (note that the outlet of the MDI 48 is further supported and stabilized by the duckbill type valve 130). It is to be understood that other types of automatic protective closure mechanisms are possible. On such alternative automatic protective closure mechanism is employed in the alternative example of Figures 49-53 described elsewhere herein.

Prior art MDI adapters, typically made of a high durometer flexible material, hold the MDI in place by engaging the entire surface of the outlet of the MDI when it is inserted therein. During insertion and removal of the MDI, the patient must exert themselves due to the considerable friction forces that are produced while sliding the MDI against the MDI adapter. Often times, this can result in the MDI adapter coming off of the associated chamber when the MDI is removed. The present disclosure, in one particular example, addresses this problem by, as seen in Figures 42, 44, and 46, providing the flexible inner portion 120 with a plurality of sliding ribs 132 extending upwardly from the inner walls 126. The sliding ribs 132 are structured to engage the outlet of an MDI, such as the outlet 54 of the MDI 48 or the outlet 58 of the MDI 50, only in limited areas, thereby reducing the friction forces that are applied to the MDI. As a result of the reduced surface contact, insertion and removal of the MDI will be easier and there will be less of a chance that the MDI adapter 118 will be pulled off of the associated main chamber, such as the main chamber housing 4.

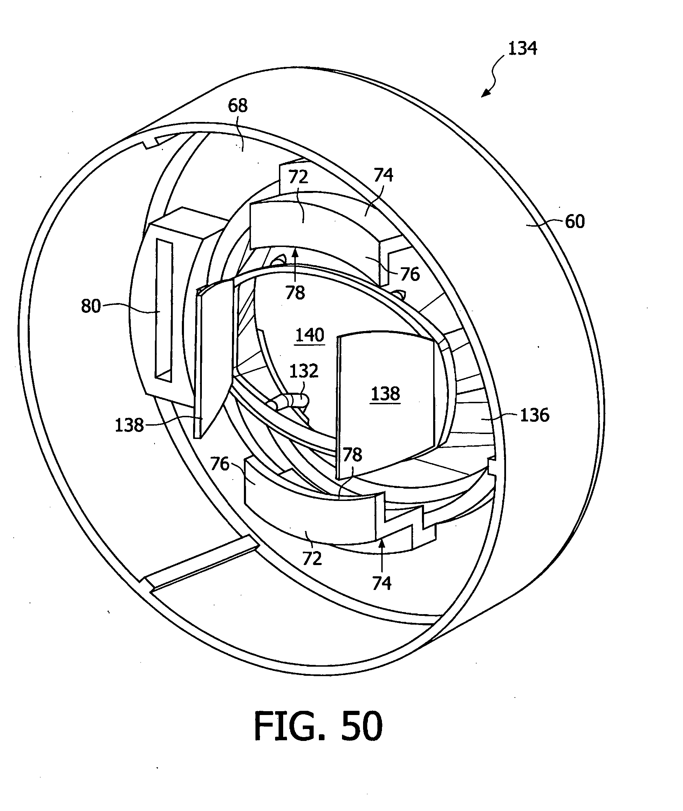

Figures 49 through 52 show various views of a two-part MDI adapter 134 according to a further alternative example which is structured to be removeably attached to the end of the main chamber housing 4 that is opposite the mouthpiece assembly 24. The MDI adapter 134 is structured to receive and hold an MDI such as MDI 48 shown in Figure 16 or MDI 50 shown in Figure 17. The MDI adapter 134 includes the rigid end cap 60 described elsewhere herein made of, for example, a hard plastic or some other suitable rigid material that is structured to be selectively attachable to the end of the main chamber housing 4 (as described elsewhere herein, the end cap 60 includes the rigid support members 72 having the arcuate surfaces 78 for supporting MDIs having round outlets such as MDI 50). The MDI adapter 134 also includes a further alternative flexible inner portion 136 made of a flexible material, such as, without limitation, silicone, rubber, TPE, or foam, among other suitable materials. The inner portion 120 is structured to be received in and held by the end cap 60 and may be made to be removable so that it can be cleaned and/or replaced if damaged, or, alternatively, may be permanently affixed to the end cap 60 by a process such as an over-molding process. The flexible inner portion 136 is similar to the flexible inner portion 118, and includes a plurality of sliding ribs 132 as described elsewhere herein. The flexible inner portion 136, like the flexible inner portion 118, includes an automatic protective closure mechanism. However, in the flexible inner portion 134, the automatic protective closure mechanism is in the form of self closing flaps 138 which are biased toward and cover aperture 140 when the MDI is not inserted in the MDI adapter 134. As illustrated in Figures 50 through 52, the self closing flaps 138 will be pushed open against the biasing force when an MDI is inserted in the MDI adapter 134.

While preferred embodiments of the invention have been described and illustrated above, it should be understood that these are exemplary of the invention and are not to be considered as limiting. Accordingly, the invention is not to be considered as limited by the foregoing description but is only limited by the scope of the appended claims.

## Claims

1. A valved holding chamber (2), comprising:
a main chamber housing (4); and
a mouthpiece assembly (24) coupled to a first end of said main chamber housing (4) **characterized in that**, said mouthpiece assembly (24) including a dual flap exhalation valve element (28) providing a one-way flow of gas from within said mouthpiece assembly (24) to ambient atmosphere, said dual flap exhalation valve element (28) comprising a first flap (42) having a first gas flow resistance and a second flap (44) having a second gas flow resistance, wherein the first resistance is less than the second resistance such that when a first exhalation gas flow having a gas flow rate that is less than or equal to a threshold gas flow rate is present within said mouthpiece assembly (24), said first flap (42) exhibits a first degree of movement as a result of said first exhalation gas flow which is greater than a second degree of movement exhibited by said second flap (44) as a result of said first exhalation gas flow.

2. The valved holding chamber (2) according to claim 1, wherein the second gas flow resistance of the second flap (44) is such that said second degree of movement is no movement.

3. The valved holding chamber (2) according to claim 1, wherein the first and second gas flow resistances are such that when a second exhalation gas flow having a gas flow rate that is greater than said threshold gas flow rate is present within said mouthpiece assembly (24), said first flap (42) exhibits a third degree of movement as a result of said second exhalation gas flow and said second flap (44) exhibits a fourth degree of movement as a result of said second exhalation gas flow, said third degree of movement and said fourth degree of movement being substantially the same.

4. The valved holding chamber (2) according to claim 1, wherein said dual flap exhalation valve element (28) comprises a single piece and wherein said first flap (42) is connected to said second flap (44).

5. The valved holding chamber (2) according to claim 4, wherein at least a portion of said first flap (42) and at least a portion of said second flap (44) are positioned directly adjacent to one another and are separated by an elongated slit.

6. The valved holding chamber (2) according to claim 1, wherein one of said first flap (42') and said second flap (44') surrounds the other of said first flap (42') and said second flap (44') on three sides thereof.

7. The valved holding chamber (2) according to claim 1, wherein said first flap (42) has a first cross-sectional area and said second flap (44) has a second cross-sectional area, said first cross-sectional area being greater than said second cross sectional area.

8. The valved holding chamber (2) according to claim 1, wherein said first flap (42) has a first thickness and said second flap (44) has a second thickness, said second thickness being greater than said first thickness.

9. The valved holding chamber (2) according to claim 1, wherein said mouthpiece (24) assembly includes a first exhalation port (36) and a second exhalation port (38), said first exhalation port (36) being larger than said second exhalation port (38), and wherein said first flap (42) is positioned to cover said first exhalation port (36) and said second flap (44) is positioned to cover said second exhalation port (38).

10. The valved holding chamber (2) according to claim 1, wherein said mouthpiece assembly (24) includes a main body portion (26) and a mouthpiece portion (30), wherein said dual flap exhalation valve element (28) is coupled to said main body portion (26), wherein said main body portion (26) and said mouth piece portion (30) are made of a rigid material, and wherein said dual flap exhalation valve element (28) is made of a flexible material.

11. The valved holding chamber (2) according to claim 1, wherein said main chamber housing (4) is generally cylindrically shaped, and wherein said mouthpiece assembly (24) includes a main body portion (26) and a mouthpiece portion (30), said main body portion (26) having a plurality of legs (32), said legs supporting said valved holding chamber (2) in a first orientation and preventing said valved holding chamber (2) from freely rolling.

12. The valved holding chamber (2) according to claim 1, further comprising an MDI adapter (46) coupled to a second end of said main chamber housing (4), said MDI adapter (46) having a rigid outer portion (60) for coupling said MDI adapter (46) to said second end of said main chamber housing (4) and a flexible inner portion (62) structured to receive and hold an outlet of an MDI (48, 50).

13. An assembly comprising a mask (92) for a respiratory drug delivery apparatus and a valved holding chamber (2) as defined in any one of claims 1 to 12, wherein a main body (94) of the mask (92) comprises a dual flap exhalation valve element (102) comprising a first flap (104) having a first gas flow resistance and a second flap (106) having a second gas flow resistance, wherein the first resistance is less than the second resistance such that when a first exhalation gas flow having a gas flow rate that is less than or equal to a threshold gas flow rate is present within said mouthpiece assembly, said first flap (104) exhibits a first degree of movement as a result of said first exhalation gas flow which is greater than a second degree of movement exhibited by said second flap (106) as a result of said first exhalation gas flow.

## Patentansprüche

1. Haltekammer mit Ventil (2), die Folgendes umfasst:
ein Hauptkammergehäuse (4); und
eine Mundstück-Baugruppe (24), die mit einem ersten Ende des genannten Hauptkammergehäuses (4) gekoppelt ist, **dadurch gekennzeichnet, dass** die genannte Mundstück-Baugruppe (24) ein Zweiklappen-Ausatemventilelement (28) umfasst, das für eine Einweg-Gasströmung aus der genannten Mundstück-Baugruppe (24) heraus in die umgebende Atmosphäre sorgt, wobei das genannte Zweiklappen-Ausatemventilelement (28) einer erste Klappe (42) mit einem ersten Gasströmungswiderstand und eine zweite Klappe (44) mit einem zweiten Gasströmungswiderstand umfasst, wobei der erste Widerstand kleiner ist als der zweite Widerstand, so dass wenn eine erste Ausatemgasströmung mit einer Gasströmungsrate, die kleiner oder gleich einer Schwellenwertgasströmungsrate ist, innerhalb der genannten Mundstück-Baugruppe (24) vorhanden ist, die genannte erste Klappe (42) einen ersten Grad an Bewegung infolge der genannten ersten Ausatemgasströmung aufweist, der größer ist als ein zweiter Grad an Bewegung, den die genannte zweite Klappe (44) infolge der genannten ersten Ausatemgasströmung aufweist.

2. Haltekammer mit Ventil (2) nach Anspruch 1, wobei der zweite Gasströmungswiderstand der zweiten Klappe (44) derartig beschaffen ist, dass der genannte zweite Grad an Bewegung keine Bewegung ist.

3. Haltekammer mit Ventil (2) nach Anspruch 1, wobei der erste und der zweite Gasströmungswiderstand derartig beschaffen sind, dass wenn eine zweite Ausatemgasströmung mit einer Gasströmungsrate, die größer als die genannte Schwellenwertgasströmungsrate ist, innerhalb der Mundstück-Baugruppe (24) vorhanden ist, die genannte erste Klappe (42) einen dritten Grad an Bewegung infolge der genannten zweiten Ausatemgasströmung aufweist und die zweite Klappe (44) einen vierten Grad an Bewegung infolge der genannten zweiten Ausatemgasströmung aufweist, wobei der genannte dritte Grad an Bewegung und der genannte vierte Grad an Bewegung im Wesentlichen gleich sind.

4. Haltekammer mit Ventil (2) nach Anspruch 1, wobei das genannte Zweiklappen-Ausatemventilelement (28) ein Einzelstück umfasst und wobei die genannte erste Klappe (42) mit der genannten zweiten Klappe (44) verbunden ist.

5. Haltekammer mit Ventil (2) nach Anspruch 4, wobei mindestens ein Teil der genannten ersten Klappe (42) und mindestens ein Teil der genannten zweiten Klappe (44) direkt nebeneinander positioniert sind und durch einen länglichen Schlitz getrennt sind.

6. Haltekammer mit Ventil (2) nach Anspruch 1, wobei entweder die genannte erste Klappe (42') oder die genannte zweite Klappe (44') die andere Klappe, also die genannte erste Klappe (42') oder die genannte zweite Klappe (44'), auf drei Seiten umgibt.

7. Haltekammer mit Ventil (2) nach Anspruch 1, wobei die genannte erste Klappe (42) eine erste Querschnittfläche hat und die genannte zweite Klappe (44) eine zweite Querschnittfläche hat, wobei die genannte erste Querschnittfläche größer ist als die genannte zweite Querschnittfläche.

8. Haltekammer mit Ventil (2) nach Anspruch 1, wobei die genannte erste Klappe (42) einer erste Dicke hat und die genannte zweite Klappe (44) eine zweite Dicke hat, wobei die genannte zweite Dicke größer ist als die genannte erste Dicke.

9. Haltekammer mit Ventil (2) nach Anspruch 1, wobei die genannte Mundstück-Baugruppe (24) eine erste Ausatemöffnung (36) und eine zweite Ausatemöffnung (38) hat, wobei die genannte erste Ausatemöffnung (36) größer ist als die genannte zweite Ausatemöffnung (38), und wobei die genannte erste Klappe (42) positioniert ist, um die genannte erste Ausatemöffnung (36) abzudecken, und die genannte zweite Klappe (44) positioniert ist, um die genannte zweite Ausatemöffnung (38) abzudecken.

10. Haltekammer mit Ventil (2) nach Anspruch 1, wobei die genannte Mundstück-Baugruppe (24) ein Hauptkörperteil (26) und ein Mundstückteil (30) enthält, und wobei das genannte Zweiklappen-Ausatemventilelement (28) mit dem genannten Hauptkörperteil (30) gekoppelt ist, wobei das genannte Hauptkörperteil (26) und das genannte Mundstückteil (30) aus starrem Material hergestellt sind, und wobei das genannte Zweiklappen-Ausatemventilelement (28) aus einem flexiblen Material besteht.

11. Haltekammer mit Ventil (2) nach Anspruch 1, wobei das genannte Hauptkammergehäuse (4) im Allgemeinen zylindrisch geformt ist, und wobei die genannte Mundstück-Baugruppe (24) ein Hauptkörperteil (26) und ein Mundstückteil (30) umfasst, wobei das genannte Hauptkörperteil (26) eine Vielzahl von Beinen (32) hat, wobei die genannten Beine die genannte Haltekammer mit Ventil (2) in einer ersten Ausrichtung unterstützen und verhindern, dass die genannte Haltekammer mit Ventil (2) frei rollt.

12. Haltekammer mit Ventil (2) nach Anspruch 1, weiterhin mit einem Dosierinhalator-Adapter (46), der mit einem zweiten Ende des genannten Hauptkammergehäuses (4) gekoppelt ist, wobei der genannte Dosierinhalator-Adapter (46) einen starren Außenteil (60) zum Koppeln des genannten Dosierinhalator-Adapters (46) mit dem genannten zweiten Ende des genannten Hauptkammergehäuses (4) und einen flexiblen Innenteil (62) mit einer Struktur zum Aufnehmen und Halten eines Auslasses eines Dosierinhalators (48, 50) umfasst.

13. Baugruppe mit einer Maske (92) für ein Gerät zur Atemwegsmedikamentverabreichung und einer Haltekammer mit Ventil (2) nach einem der Ansprüche 1 bis 12, wobei ein Hauptkörper (94) der Maske (92) ein Zweiklappen-Ausatemventilelement (102) mit einer ersten Klappe (104) mit einem ersten Gasströmungswiderstand und eine zweite Klappe (106) mit einem zweiten Gasströmungswiderstand umfasst, wobei der erste Widerstand kleiner ist als der zweite Widerstand, so dass wenn eine erste Ausatemgasströmung mit einer Gasströmungsrate, die kleiner oder gleich einer Schwellenwertgasströmungsrate ist, innerhalb der genannten Mundstück-Baugruppe vorhanden ist, die genannte erste Klappe (104) einen ersten Grad an Bewegung infolge der genannten ersten Ausatemgasströmung aufweist, der größer ist als ein zweiter Grad an Bewegung, den die genannte zweite Klappe (106) infolge der genannten ersten Ausatemgasströmung aufweist.

## Revendications

1. Chambre de retenue à valve (2), comprenant :
un logement de chambre principal (4) ; et
un ensemble embout buccal (24) accouplé à une première extrémité dudit logement de chambre principal (4), **caractérisé en ce que** ledit ensemble embout buccal (24) comprend un élément valve d'expiration à deux clapets (28) fournissant un écoulement unidirectionnel de gaz de l'intérieur dudit ensemble embout buccal (24) à l'atmosphère ambiante, ledit élément valve d'expiration à deux clapets (28) comprenant un premier clapet (42) possédant une première résistance à l'écoulement de gaz et un second clapet (44) possédant une seconde résistance à l'écoulement de gaz, dans laquelle la première résistance est inférieure à la seconde résistance de sorte que, lorsqu'un premier écoulement de gaz d'expiration possédant un débit de gaz qui est inférieur ou égal à un débit de gaz de seuil est présent à l'intérieur dudit ensemble embout buccal (24), ledit premier clapet (42) présente un premier degré de mouvement en conséquence dudit premier écoulement de gaz d'expiration, qui est supérieur à un deuxième degré de mouvement présenté par ledit second clapet (44) en conséquence dudit premier écoulement de gaz d'expiration.

2. Chambre de retenue à valve (2) selon la revendication 1, dans laquelle la seconde résistance à l'écoulement de gaz du second clapet (44) est telle que ledit deuxième degré de mouvement soit un mouvement nul.

3. Chambre de retenue à valve (2) selon la revendication 1, dans laquelle les première et seconde résistances à l'écoulement de gaz sont telles que, lorsqu'un second écoulement de gaz d'expiration possédant un débit de gaz qui est supérieur audit débit de gaz de seuil est présent à l'intérieur dudit ensemble embout buccal (24), ledit premier clapet (42) présente un troisième degré de mouvement en conséquence dudit second écoulement de gaz d'expiration et ledit second clapet (44) présente un quatrième degré de mouvement en conséquence dudit second écoulement de gaz d'expiration, ledit troisième degré de mouvement et ledit quatrième degré de mouvement étant sensiblement les mêmes.

4. Chambre de retenue à valve (2) selon la revendication 1, dans laquelle ledit élément valve d'expiration à deux clapets (28) comprend une seule pièce et dans laquelle ledit premier clapet (42) est raccordé audit second clapet (44).

5. Chambre de retenue à valve (2) selon la revendication 4, dans laquelle au moins une partie dudit premier clapet (42) et au moins une partie dudit second clapet (44) sont positionnées de façon directement adjacente l'une à l'autre et sont séparées par une fente allongée.

6. Chambre de retenue à valve (2) selon la revendication 1, dans laquelle un parmi ledit premier clapet (42') et ledit second clapet (44') entoure l'autre parmi ledit premier clapet (42') et ledit second clapet (44') sur trois côtés de celui-ci.

7. Chambre de retenue à valve (2) selon la revendication 1, dans laquelle ledit premier clapet (42) possède une première superficie de section transversale et ledit second clapet (44) possède une seconde superficie de section transversale, ladite première superficie de section transversale étant supérieure à ladite seconde superficie de section transversale.

8. Chambre de retenue à valve (2) selon la revendication 1, dans laquelle ledit premier clapet (42) possède une première épaisseur et ledit second clapet (44) possède une seconde épaisseur, ladite seconde épaisseur étant supérieure à ladite première épaisseur.

9. Chambre de retenue à valve (2) selon la revendication 1, dans laquelle ledit ensemble embout buccal (24) comprend un premier orifice d'expiration (36) et un second orifice d'expiration (38), ledit premier orifice d'expiration (36) étant plus grand que ledit second orifice d'expiration (38), et dans laquelle ledit premier clapet (42) est positionné pour couvrir ledit premier orifice d'expiration (36) et ledit second clapet (44) est positionné pour couvrir ledit second orifice d'expiration (38).

10. Chambre de retenue à valve (2) selon la revendication 1, dans laquelle ledit ensemble embout buccal (24) comprend une partie corps principal (26) et une partie embout buccal (30), dans laquelle ledit élément valve d'expiration à deux clapets (28) est accouplé à ladite partie corps principal (26), dans laquelle ladite partie corps principal (26) et ladite partie embout buccal (30) sont faites d'un matériau rigide, et dans laquelle ledit élément valve d'expiration à deux clapets (28) est fait d'un matériau flexible.

11. Chambre de retenue à valve (2) selon la revendication 1, dans laquelle ledit logement de chambre principal (4) présente une forme généralement cylindrique, et dans laquelle ledit ensemble embout buccal (24) comprend une partie corps principal (26) et une partie embout buccal (30), ladite partie corps principal (26) possédant une pluralité de pieds (32), lesdits pieds supportant ladite chambre de retenue à valve (2) dans une première orientation et empêchant ladite chambre de retenue à valve (2) de rouler librement.

12. Chambre de retenue à valve (2) selon la revendication 1, comprenant en outre un adaptateur MDI (46) accouplé à une seconde extrémité dudit logement de chambre principal (4), ledit adaptateur MDI (46) possédant une partie extérieure rigide (60) pour accoupler ledit adaptateur MDI (46) à ladite seconde extrémité dudit logement de chambre principal (4) et une partie intérieure flexible (62) structurée pour recevoir et retenir une sortie d'un MDI (48, 50).

13. Ensemble comprenant un masque (92) pour un appareil d'administration de médicament respiratoire et une chambre de retenue à valve (2) selon une quelconque des revendications 1 à 12, dans lequel un corps principal (94) du masque (92) comprend un élément valve d'expiration à deux clapets (102) comprenant un premier clapet (104) possédant une première résistance à l'écoulement de gaz et un second clapet (106) possédant une seconde résistance à l'écoulement de gaz, dans lequel la première résistance est inférieure à la seconde résistance de sorte que, lorsqu'un premier écoulement de gaz d'expiration possédant un débit de gaz qui est inférieur ou égal à un débit de gaz de seuil est présent à l'intérieur dudit ensemble embout buccal, ledit premier clapet (104) présente un premier degré de mouvement en conséquence dudit premier écoulement de gaz d'expiration, qui est supérieur à un deuxième degré de mouvement présenté par ledit second clapet (106) en conséquence dudit premier écoulement de gaz d'expiration.
